# EUROPEAN PATENT APPLICATION

(11) **EP 4 289 282 A1**
(43) Date of publication of application: **13.12.2023**
(21) Application number: 21924867.1
(22) Date of filing: 23.12.2021
(51) Int. Cl.: A23J 3/00, A23J 3/14

(54) **PSEUDO-MEAT FOOD PRODUCT AND METHOD FOR PRODUCING PSEUDO-MEAT FOOD PRODUCT**

(30) Priority: 08.02.2021 JP 2021018423; 24.09.2021 JP 2021155609
(71) Applicant: Banseisha Co., Ltd., Yokohama-shi, Kanagawa 231-0023 (JP)
(72) Inventor: SUNG, Youngsoon, Yokohama-shi, Kanagawa 231-0023 (JP)
(74) Representative: Horn Kleimann Waitzhofer Schmid-Dreyer Patent- und Rechtsanwälte PartG mbB
(86) International application number: PCT/JP2021/047897
(87) International publication number: WO 2022/168482

(57) **Abstract**

Provided is an imitation-meat food that causes little environmental impact, uses a highly nutritious hemp protein source, and can provide a flavor and texture similar to those of natural meat when chewed. This imitation-meat food contains a vegetable protein source as its principal ingredient. The vegetable protein source is constituted from a mixture of a hemp protein source made up of hemp seeds and/or hemp nuts, and powdered quinoa mixed in so as to be at a predetermined proportion with respect to the hemp protein source.

## Description

### TECHNICAL FIELD

The present invention affords an imitation-meat food product, and a method of fabricating an imitation-meat food product, that may be exploited as a meat substitute.

### BACKGROUND ART

Imitation-meat food-product materials, at one time developed as an inexpensive substitute for animal protein, today are once again attracting attention as low-calorie foodstuffs or as livestock-meat substitute material for patients with diseases due to which their intake of animal protein is restricted. An example of conventional art that relates to imitation-meat food products is the meat-like food product disclosed in Patent Document 1.

### PRECEDENT TECHNICAL LITERATURE

### Patent Document(s)

**Patent Document 1:** Japanese Unexamined Pat. App. Pub. No. S60-156345

### SUMMARY OF INVENTION

### Issues Claimed Subject Matter Is to Address

Nevertheless, there have been problems with the imitation-meat food product set forth in Patent Document 1, which is fabricated from a vegetable protein in the form of a mass, a vegetable protein in the form of fibers, and a binder, in that although table meat is reproduced with the vegetable protein, the flavor is meager, and the mouthfeel is unnatural.

In a similar way, as imitation-meat food products utilizing vegetable proteins, products exploiting soybeans have gained much currency. Yet imitation meat utilizing soybeans and processed foods from soybeans is dominated by the flavor peculiar to soybeans and tastes bland, which is prohibitive of sensing the richness and sweetness characteristic of meat. Moreover, what with the chewing sensation not lending a sense of a meat-like mouthfeel, the unnaturalness cannot be gotten rid of. What is more, there are issues from a nutritional-value aspect, in that while imitation meat utilizing soybeans and processed foods from soybeans may have a high protein content, it does not have any more nutritional value than that of soybeans. The same things can be said about imitation-meat food products exploiting broad beans.

Against that backdrop, instead of soybeans or broad beans as the vegetable protein to be the principal ingredient of imitation-meat food products, the inventor(s) set her sights on hemp protein sources in which hemp seeds and hemp nuts-hemp-plant hearts-are rendered into powdered form, and set about developing an imitation-meat food.

Hemp seeds and hemp nuts, rich not only in protein, but also in essential amino acids and minerals, encompass the entirety of food-meat nutrients that cannot be reproduced with imitation-meat food products using soybeans or broad beans, enabling a total substitute for table meat.

And whereas the manufacture of table meat is obviously destroying the global environment, hemp is a plant whose cultivation is easy such that, provided that a variety with no stimulant effects is chosen, it can be readily grown in colonies even in mountains, wherein inasmuch as it absorbs carbon dioxide and releases oxygen, it lessens the burden on the environment, facilitating the acquisition of total protein of high nutritional value.

Nevertheless, although hemp is easy to come by and is of high nutritional value, given that hemp has a characteristic grassy odor, bitterness, and astringency, using naive hemp-derived vegetable protein to create an imitation-meat food possessing the flavors of table meat is challenging. And in that viscosity and elasticity are absent from hemp, there has been room for further betterment in order to obtain a meat-like mouthfeel.

Therein, an object of the present invention is to make available an imitation-meat food, and a method for fabricating an imitation-meat food, that exploit a hemp protein source whose burden on the environment is minimal and that is of high nutritional value, and that when chewed can yield flavors and mouthfeel near that of natural meat.

### Means for Resolving the Issues

The inventor(s) discovered as a result of intensive research that the issues noted above can be resolved by mixing quinoa powder in with a hemp protein source, which led to the present invention.

An imitation-meat food involving a first feature is an imitation-meat food with a vegetable protein source being the principal ingredient, wherein the vegetable protein source is constituted from a mixture of a hemp protein source made up of hemp seeds and/or hemp nuts, and powdered quinoa mixed in so as to be at a predetermined proportion with respect to the hemp protein source.

According to the invention involving the first feature, mixing in powdered quinoa in a predetermined proportion with respect to the hemp protein source compensates for the bitterness and meagerness of mouthfeel peculiar to hemp, with the elasticity and flavors that quinoa possesses, enabling an imitation-meat food that has a meat-like elasticity and in which hemp-derived bitterness is scarce to be made available.

An imitation-meat food involving a second feature is the invention involving the first feature, wherein the proportion of powdered quinoa mixed in with the hemp protein source is determined based on foodstuff data in which the taste, smell, and mouthfeel of created imitation-meat food have been converted into numerical values.

According to the invention involving the second feature, the fact that the proportion of powdered quinoa mixed in with the hemp protein source is determined based on foodstuff data in which the taste, smell, and mouthfeel of created imitation-meat food have been converted into numerical values deters expression of excessive stickiness and excessive sweetness from the mixing in of quinoa protein, enabling an imitation-meat food possessing just the right elasticity and flavors to be made available.

### ADVANTAGEOUS EFFECTS OF INVENTION

According to the present invention an imitation-meat food, and a method of its production, in which a hemp protein source whose burden on the environment is minimal and that is of high nutritional value is exploited, and that when chewed can yield flavors and mouthfeel near that of natural meat may be made available.

### BRIEF DESCRIPTION OF THE DRAWINGS

Fig. 1 is an explanatory chart presenting an outline of a method of fabricating an imitation-meat food of the present invention.
Fig. 2 is an explanatory diagram illustrating an overview of an apparatus for mold-processing imitation meat in the method of fabricating an imitation-meat food of the present invention.
Fig. 3 is an explanatory diagram illustrating an imitation-meat molding process in the method of fabricating an imitation-meat food of the present invention.
Fig. 4 is an explanatory diagram illustrating an imitation-meat molding process in the method of fabricating an imitation-meat food of the present invention.
Fig. 5 is an explanatory diagram illustrating an imitation-meat molding process in the method of fabricating an imitation-meat food of the present invention.
Fig. 6 is an explanatory diagram illustrating an imitation-meat molding process in the method of fabricating an imitation-meat food of the present invention.
Fig. 7 is an explanatory diagram illustrating an imitation-meat molding process in the method of fabricating an imitation-meat food of the present invention.
Fig. 8 is an explanatory diagram illustrating an imitation-meat molding process in the method of fabricating an imitation-meat food of the present invention.
Fig. 9 is an explanatory diagram illustrating an imitation-meat molding process in the method of fabricating an imitation-meat food of the present invention.

### MODES FOR EMBODYING THE INVENTION

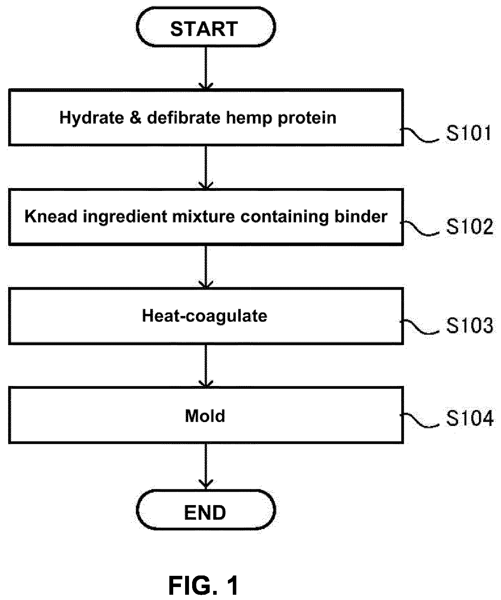

In the following, referring to the accompanying drawings, an explanation of modes of embodying methods of fabricating imitation-meat food involving the present invention will be made in detail. Fig. 1 is an explanatory chart presenting an outline of a method of fabricating an imitation-meat food involving a first embodying mode. It should be noted that the process regimen that is explained in the following is nothing more than a single example; each process may be altered within the limits of possibility. Also, as to the process regimen that is explained in the following, according to the embodying mode, the omission, substitution, or addition of steps as appropriate is possible.

### Imitation-Meat Food Product Involving a First Embodying Mode

To begin with, a method of fabricating, and an apparatus for fabricating, an imitation-meat food product involving a first embodying mode will be described.

### Method of Fabricating Imitation-Meat Food Product Involving First Embodying Mode

A method of fabricating an imitation-meat food product involving a first embodying mode includes, as indicated in Fig. 1: a process of hydrating and defibrating a vegetable protein ingredient largely containing a hemp protein source, together with starch, to create a texturized vegetable protein (Step S101); a process of coagulating an ingredient mixture containing the created texturized vegetable protein and a binder (Step S102 and Step S103); and a process of molding it into a food of predetermined form (Step S104). Each process will be elaborated below.
(1) Texturized-Vegetable-Protein Creating Process

In the texturized-vegetable-protein creating process (S101), a vegetable protein containing a hemp protein source is, together with starch, hydrated and defibrated to create a texturized vegetable protein. The first embodying mode includes, with this texturized-vegetable-protein creating process, fibrous matter prepared by hydrating and defibrating vegetable protein containing a hemp protein source. Specifically, as the hemp protein source, in the first embodying mode powdered hemp in which hemp seed (hemp-plant hearts) has been pulverized into powder form is utilized. This hemp seed, as a hemp protein resource, contains abundant protein and fibrous matter, with the protein containing twenty amino acids, included among which are the nine amino acids that are known as "the essential amino acids."

Next, the powdered hemp, being the vegetable protein obtained in this way, together with starch is hydrated by being caused take in water, and according as necessary, excess water content is removed. The hydrated matter is thereafter defibrated, that is, loosened into fibrous matter. Hydration can be carried out by soaking dried granular vegetable protein in water or warm water. Herein, as the starch, substances that are pulverized potatoes, nuts, brown rice or other grains, or edible kernels of chestnuts or acorns, or the like are utilized. And at this point, mushrooms (shiitake, king oyster mushrooms) defibrated to render them into fibrous matter may be compounded in.

It should be noted that in the above-described process of creating texturized vegetable protein, a process of extracting with an aqueous calcium-salt solution the powdered hemp obtained by grinding hemp seed, and of solubilizing the hemp protein from the protein source to form an aqueous hemp-protein solution, and a process of at least partially separating the aqueous hemp protein solution from the residual hemp protein source may be implemented. The texturized-vegetable-protein creating process may be designed so that the separated aqueous hemp-protein solution is then concentrated, and the concentrated hemp-protein solution is dried.

As the vegetable protein, besides a hemp protein source, soy protein, wheat protein, and the like can be utilized combined with a hemp protein source. As the soy protein, defatted soy flour may be utilized, or powdered soy protein (isolated soy protein) may be utilized.

Herein, a mixture containing at least the vegetable protein ingredient and water-and as required, further containing fats and oils, starch, pigments, flavorings, etc. is pressurize-heated to bulk it. This bulked matter has a fibrous internal structure possessing regular directionality, wherein it has been so-called texturized. The obtained bulked matter is then cut into suitable sizes and dried, creating texturized vegetable protein.

Compounded-in are, for example, beet powder, salt, calcium, olive oil, in addition to which are vegetable (including cabbage, potato, *shiitake,* king-oyster-mushroom, daikon, or onion) soup, and radish *siraegi* (dried radish greens).

The texturized vegetable protein described above can be utilized having been colored. Coloring enables an imitation-meat food product that is close to natural meat in outward appearance to be made available. For example, when beef is mimicked, some any of it may be rendered in a red, and the rest rendered in a white; specifically, rendering the texturized vegetable protein in a white and rendering the fibrous vegetable protein in a red enables an imitation-meat food product that is close to natural beef to be made available.

### (2) Ingredient-Mixture Creating Process

Next, a process (S102) in which the texturized vegetable protein created in the above-described texturized-vegetable-protein creating process is kneaded together with a binder to create an ingredient mixture is carried out.

In the first embodying mode, it is preferable that the ingredient mixture be compounded with starch, gluten, and lipids. It will be appreciated that as the starch, gluten, and lipids referred to herein, substances that are pulverized potatoes, nuts, brown rice or other grains, or edible kernels of chestnuts or acorns, or the like can be utilized.

In the process in which the ingredient mixture is coagulated, the binder can be one or more selected from the group consisting of vegetable proteins, animal proteins, and polysaccharides. And at least a portion of the binder can be a binding agent that constitutes a thermoreversible gel on being hydrated, wherein it can be contained in the form of a thermoreversible gel in which the ingredient mixture has hydrated the binder.

Furthermore, the process of coagulating the ingredient mixture can include: a process of varying the formulation of the ingredient mixture to create a plurality of mouthfeel fundamentals that furnish differing mouthfeels; a process of heating or cooling the plurality of created mouthfeel fundamentals to coagulate them; and a process of molding the coagulated ingredient mixture.

The vegetable protein utilized as the binder herein may be, to cite some examples, soy protein or wheat protein; the animal protein may be, to cite some examples, egg white, collagen, or milk protein; and the polysaccharide may be, to cite some examples, starch, guar gum or other gums, carrageenan, and glucomannan or other gellant. Also, the binder may be in the form of a paste in which it is has been kneaded together with water or fats/oils and added to the ingredient mixture. Furthermore, the binder preferably is added to the ingredient mixture in the form of an emulsion in which it is has been emulsified together with water and fats/oils. In this embodying mode, the emulsion forms a fat-like texture within the imitation-meat composition, thus imparting to it a look and mouthfeel (the juicy feel of fatty meat) that are still nearer to those of meat. As the fats/oils, any among edible vegetable fats/oils including soybean oil, cottonseed oil, corn oil, and sesame oil, and edible animal fats/oils including beef tallow, lard, chicken fat, and butter can be employed.

Furthermore, as at least a portion of the binder, a binding agent that constitutes a thermoreversible gel on being hydrated can be employed. In that case, an ingredient mixture in which the binding agent has been hydrated in advance to constitute a thermoreversible gel, and the constituted thermoreversible gel has been mixed together with other ingredients is used for a shaping-heating step. A "thermoreversible gel" refers to a gel from a substance that liquefies when heated and congeals when cooled. In this embodying mode, thanks to the thermoreversible gel forming a fat-like texture within the imitation-meat composition, it is imparted with a look and mouthfeel (the juicy feel of fatty meat) that are still nearer to those of meat. The binding agent that constitutes a thermoreversible gel on being hydrated may be, to cite some examples, carrageenan, glucomannan, or agar. In order to constitute the gel, water at, by mass, 40 to 100 times the amount may, for example, be added to these binding agents, which may be suitably heated, stirred, and cooled as required.

The ingredient mixture may further be compounded with a protein-binding enzymes such as a transglutaminase. Compounding-in a protein-binding enzyme strengthens the binding between the fibers of the texturized vegetable protein and the fibers of the fibrous vegetable protein, and between these fibers and the binding agent, yielding a natural meat-like tissue, and fortifying a mouthfeel in which there is a natural meat-like chewing response.

The ingredient mixture may also contain, among other things, water, fats/oils, flavorings, condiments (including meat/poultry/seafood extracts), starch, and pigments. In particular, flavorings for imparting the taste of livestock meats such as beef, pork, or chicken, or meat/poultry/ seafood extracts, or the like are preferably added. As fats/oils, substances similar to what has been described above with regard to the binding agent may be employed.

It will be appreciated that for the ingredient mixture, different conditions including the compounding ratios of vegetable protein, water, and other ingredients, and the pressure and temperature, are appropriately selected according to the intended mouthfeel. The form of the texturized vegetable protein post-drying may be, to cite some examples, granular, rodlike, or flaky, but is not particularly limited. Dried texturized vegetable protein, as well as texturized vegetable protein having been shaped into different forms, are both comprehended by granular vegetable protein.

### (3) Heat-Coagulation Step and Shaping Step

Next, the ingredient mixture created in the ingredient-mixture creating process (S102) is coagulated (S103). In the first embodying mode, the process of coagulating the ingredient mixture includes: a process of varying the formulation of the ingredient mixture to create a plurality of foodstuff fundamentals that furnish differing tastes and mouthfeels; and a process of heating or cooling the plurality of created mouthfeel fundamentals to coagulate them. After these, a molding step (S104) is carried out.

It will be appreciated that the ingredient mixture is shaped into a desired form (forms including, for example, sliced-meat forms, block-meat forms, or forms in which it is cube-chunked). Shaping can be carried out utilizing appropriate molds. Heating the molded ingredient mixture at a temperature of between 60°C and 135°C, preferably between 70°C and 120°C, coagulates the molded product to yield an imitation-meat food product. Preferably the ingredient-mixture molded product is heated and coagulated in a state in which it has been sealed inside a matrix for molding or inside a container such as a retort pouch. The form is not limited to mold forms, the point being that the obtained imitation-meat food product should take on the desired form, wherein a method such as casting the ingredient mixture into hot water may be adopted.

### Food-Product Fabrication apparatus for Imitation-Meat Food Product Involving First Embodying Mode

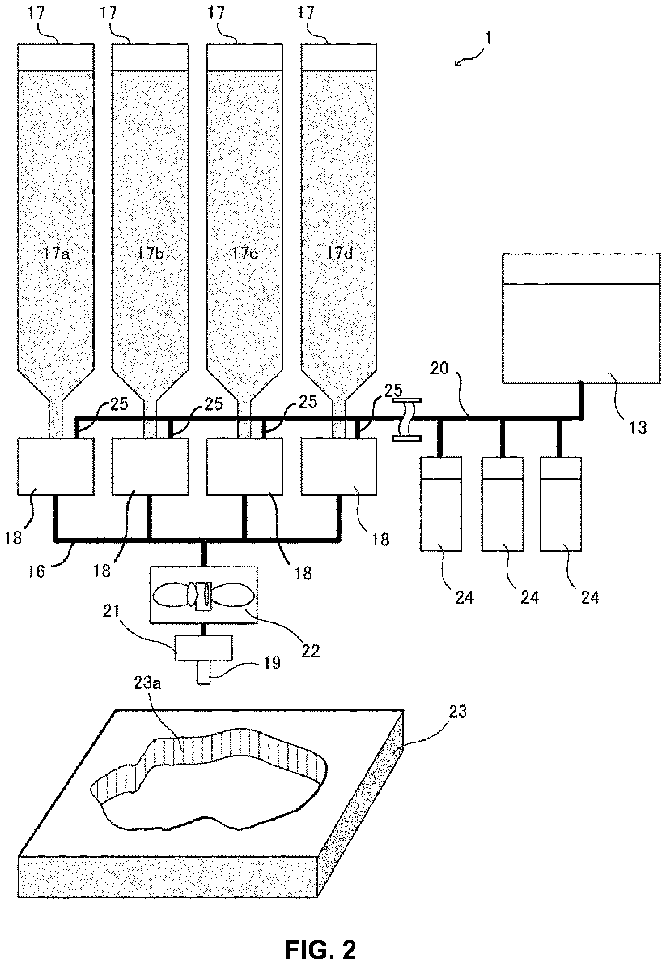

Next, referring to Fig. 2 to 9, a description of the configuration and the operation of a food-product fabrication apparatus involving the first embodying mode will be made. Fig. 2 is an overview diagram of a food-product fabrication apparatus 1 involving the first embodying mode. In the first embodying mode, the above-described heat-coagulation step and the shaping step (Steps S103 and S104) are carried out utilizing the food-product fabrication apparatus 1, wherein in the course of kneading and heat-coagulating the different types of foodstuffs, on the basis of three-dimensional form data a three-dimensional shaped article is formed, and the complex three-dimensional structure of the food product is reproduced with a 3D printer.

In particular, in the first embodying mode, a predetermined food product is databased; more specifically, its taste, color, shape, and mouthfeel are retained as foodstuff data converted into numerical values, and the foodstuff data, combined with a plurality of mouthfeel fundamentals that furnish differing mouthfeels, is converted into food-product reproduction data relating to constituent ratios of the plurality of mouthfeel fundamentals for reproducing the desired taste, color, shape, and mouthfeel. Next, a mixture in which plural fundamentals have been mixed in predetermined proportions is obtained and the mixture is heated or cooled, thereby reproducing based on the foodstuff data the desired taste, color, shape, and mouthfeel. The mixture is then molded to fabricate a food product of a desired, compositive form.

To begin with, a schematic configuration of the food-product fabrication apparatus 1 overall will be described. As illustrated in Fig. 2, the food-product fabrication apparatus 1 is constituted by putting into place a water tank 13, foodstuff tanks 17, mixing means 18, and an output nozzle 19.

The water tank 13 is in the form of a bottomed round cylinder, and stores water in its interior. Connected to the bottom part of the water tank 13 is a main pipe 20, extending directed downward, the tip (lower end) of which is ultimately connected to the output nozzle 19. The water tank 13 is outfitted with a computer (not illustrated) that constitutes control means and communications means.

The foodstuff tanks 17 contain fundamentals that differ from each other in mouthfeel, color, and taste. Specifically, four fundamentals 17a - d are ingredient mixtures of plural kinds in which hemp protein and another foodstuff are mixed, such as, for example: a first essential with hemp and rice being the principal ingredients; a second essential with hemp and seaweed being the principal ingredients; a third essential with hemp and beans being the principal ingredients; and a fourth essential with hemp and konjac being the principal ingredients.

Further, to the lower ends of the foodstuff tanks 17, the mixing means 18 are respectively connected. Predetermined quantities of the mouthfeel fundamentals in each foodstuff tank 17 are delivered by a dispenser being controlled according to commands from the not-illustrated control unit, and are sent from the lower ends to the respective mixing means 18.

Each mixing means 18 creates a mixture obtained by kneading fundamentals delivered from the above-described foodstuff tanks 17 together with water supplied by way of the main pipe 20. Specifically, the mixing means 18 are furnished with stirring means that mix together and stir the above-described fundamentals and water. In addition to creating the mixtures, the mixing means 18 also serve as mouthfeel-reproducing means that by heating or cooling the mixtures reproduce, based on mouthfeel data, desired mouthfeels.

The mixing means 18 stir so as to be uniform the above-described fundamentals and water supplied by way of the main pipe 20, creating the mixtures. Further, the mixing means 18 also function to heat-process the mixtures by indirect boiling or the like. The mixing means 18 are also provided with a cooling means such as a cooling fan underneath the mixing means 18, wherein it cools mixtures that have been stirred and heated by the mixing means 18. This enables mixtures coagulated into gel form to be obtained.

To the underneath of all the mixing means 18, an extruding means 21 is connected via the stirring means 22, and to the underneath of the extruding means 21, the output nozzle 19 is connected. The output nozzle 19 is shiftably supported by a movable arm that is shiftable horizontally and vertically in triaxial (*x*-axial, *y*-axial, and *z*-axial) directions, wherein while shifting along the three axes (*x*-axis, *y*-axis, and z-axis), it intermittently extrudes from its tip mixtures having gone through the mixing means 18, whereby the mixtures are molded into predetermined forms.

Underneath the output nozzle 19, a die frame 23 is disposed. In the die frame 23, the outer form of the food product that will be the subject of molding is chiseled out as recesses 23a, and by the extruding into the recesses 23a of mixture from the tip of the output nozzle 19 and causing of movement of the output nozzle 19 along the three axes (x-axis, y-axis, and z-axis) on the basis of commands from the control unit, new imitation meat 3 that has been shaped into a predetermined form can be fabricated.

Moreover, midway along the main pipe 20 connected to the water tank 13, three enzyme cartridges 24 for adjusting the mouthfeel of the mixtures are connected. The different enzyme cartridges 24 house enzymes for reproducing differing tastes and mouthfeels. The enzymes include, to cite examples, transglutaminase (TG) for reproducing firmness in the imitation meat 3, α-glucosidase (AG) for reproducing a moist mouthfeel in the imitation meat 3, and protein glutaminase (PG) for reproducing a smooth mouthfeel in the imitation meat 3. It should be understood that the enzymes are not limited to these, and suitable modifications and additions are possible.

Each enzyme cartridge 24 is connected to the main pipe 20 via a dispenser or other metered liquid-administering device. Then by the dispenser being controlled by the control unit, predetermined quantities of enzyme are sent to the main pipe 20. Downstream ends 25 of the main pipe 20 are respectively connected to the mixing means 18 via water inlets through which water mixed with predetermined quantities of enzymes is injected.

Provided between a tube 16 and the output nozzle 19 are: the mixing means 18 as mixing means for mixing together the fundamentals 17a - d, water, and enzymes to create mixtures; the stirring means 22 as reproducing means for heating or cooling the mixtures to reproduce based on the foodstuff data desired tastes, colors, and mouthfeels; and the extruding means 21.

### Operation of Food-Product Fabrication apparatus for Imitation-Meat Food Product Involving First Embodying Mode

By operating the food-product fabrication apparatus 1 described in the foregoing, a method of fabricating an imitation-meat food product involving the first embodying mode can be carried out. A production method involving the first embodying mode is a method of fabricating imitation meat 3 on the basis of foodstuff data in which the taste, color, shape, and mouthfeel of imitation meat have been converted into numerical values, wherein it specifically includes steps of, on the basis of the foodstuff data, mixing, heat-coagulating, and shaping an ingredient mixture in which a texturized vegetable protein has been compounded. In the first embodying mode, the steps of mixing, heat-coagulating, and forming the ingredient mixture on the basis of foodstuff data are carried out with the above-described food-product fabrication apparatus.

To elaborate: a mixture is obtained in which are mixed, in predetermined proportions, water, enzymes, and the fundamentals 17a-d, which are ingredient mixtures in which plural texturized vegetable proteins have been compounded on the basis of foodstuff reproduction data. Specifically, the food-product fabrication apparatus 1 controls the dispenser or the like on each cartridge on the basis of the foodstuff reproduction data. In addition, the food-product fabrication apparatus 1, by means of the mixing means 18, mixes the fundamentals 17a-d and the enzymes supplied from each cartridge in predetermined quantities, with water supplied in a predetermined quantity from the water tank 13 to create a mixture of these.

Next, a heat-coagulation step by means of heating or cooling is performed. Herein, heating or cooling the mixture reproduces desired taste, color, and mouthfeel based on the foodstuff data. Specifically, the mixing means 18 heats the mixture by indirect boiling or the like, or by directing a cold gust on the mixture with a cooling fan or the like, implementing heat-processing on the mixture to reproduce a desired mouthfeel.

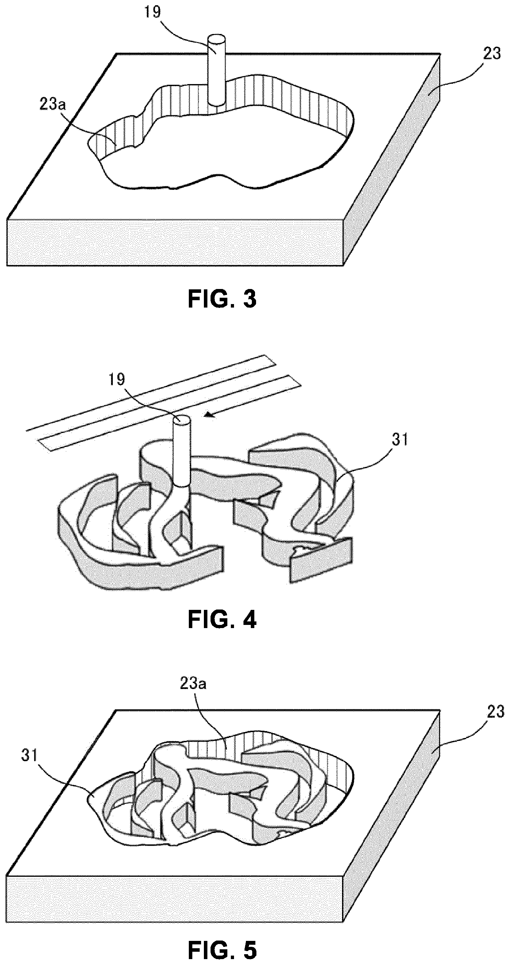

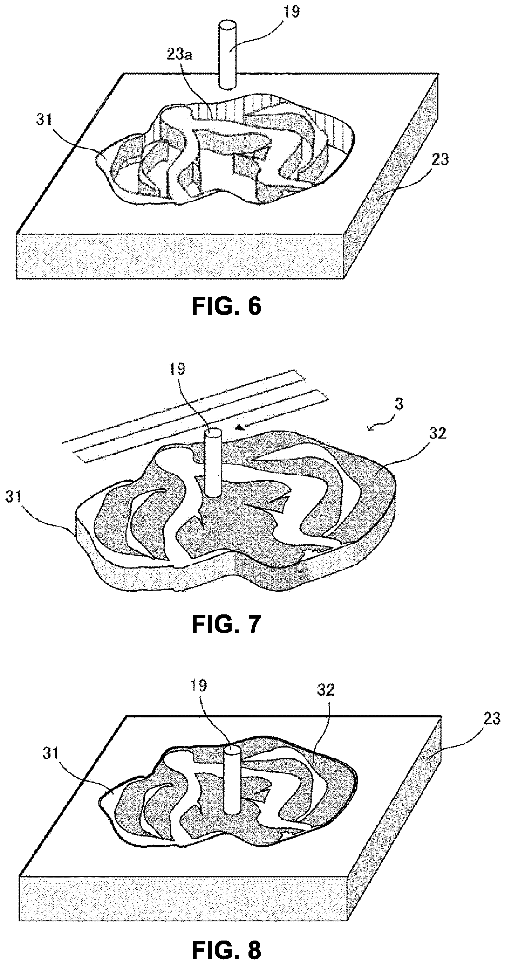

Next, a shaping step is performed. In the molding step, as illustrated in Figs. 3 through 5, inside the die frame 23, meat fatty areas 31, for example, are sectionally molded from a predetermined mixture, and meanwhile into the remaining areas, as illustrated in Figs. 6 through 8, a mixture with the taste, color, and mouthfeel of what will be meat lean areas 32 is poured in, fabricating imitation meat 3 of predetermined form. Specifically, the food-product fabrication apparatus 1 plots out the mixture from the output nozzle 19 onto the die frame 23. Various methods of plotting out the mixture are possible.

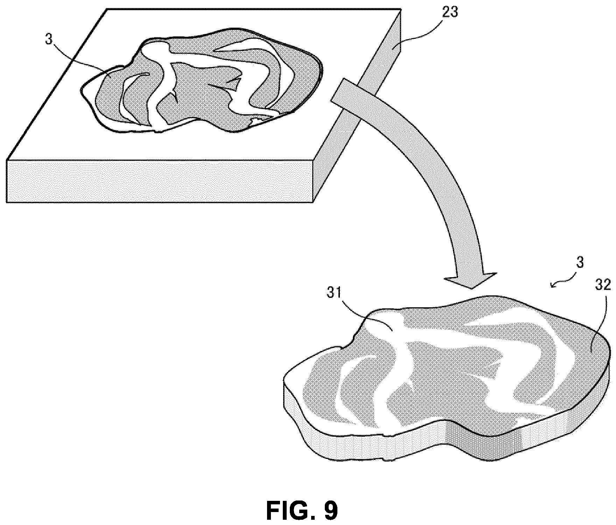

The food-product fabrication apparatus 1 is, for example, enabled to plot out a mixture in gel form in points or lines so as to be of predetermined thickness in the form of squares in plan view, and to repeat this multiple times, stacking the mixture in the form of plural layers to mold it into a predetermined form. Also, a mixture in gel form may be shaped into a predetermined form by injecting it into the recesses 23a on the die frame 23 up to a predetermined depth and hardening it. By in this way appropriately changing the plotting method in accordance with the data structure of predetermined food products to be reproduced, new imitation meat 3 in which the mouthfeel of predetermined food products of all sorts has been reproduced including for example that of imitation meat 3, as illustrated in Fig. 9, in which meat fatty areas 31 are disposed among meat lean areas 32-can be molded into predetermined forms.

It should be noted that it is not necessary that the above-described mixing step, heating/cooling step, and shaping step be performed in that order; each step may be carried out together in consecutive flows, or the order may be switched around. For example, after shaping it into cubic forms, heating/cooling may be performed, and the mixture hardened. In that case, the heating/cooling step shoulders a portion of the shaping step.

### Action and Effect of Invention Involving First Embodying Mode

According to the invention involving the first embodying mode, effective advantage is taken of a hemp protein source, whereby as indicated in Table 1 and Table 2, the nutritional value even compared with meat per se is high, and moreover, flavor and mouthfeel close to that of natural meat can be obtained in appearance as well, an imitation-meat food product that is close to natural meat can be obtained.

To elaborate: as indicated in the same tables, the hemp protein source contained in hemp meat, compared with other vegetable source materials such as soybeans and broad beans, contains in good balance nutritional content equivalent to that of animal protein, wherein it proves to be a total substitute for animal protein. Animal protein has a large impact on the global environment in view of the amount of water and oxygen used for, as well as the amount of carbon dioxide emitted by, livestock farming, whereas hemp protein resources, given their ease of cultivation and the fact that, thanks to being plant-derived, they absorb carbon dioxide and release oxygen, have a minimal burden on the environment, facilitating the acquisition of complete proteins.

**Table 1**

| | **Wagyu/Fillet/Red Meat** | **Hemp Meat** | |
|---|---|---|---|
| Proteins | 19.1 | 26.26 | g |
| Fats | 15 | 16.56 | g |
| Carbohydrates | 0.3 | 22.91 | g |
| Salt Equivalent | 0.1 | 0.64 | g |
| Saturated Fatty Acids | 5.79 | 1.25 | g |
| Monounsaturated Fatty Acids | 6.9 | 1.55 | g |
| Polyunsaturated Fatty Acids | 0.49 | 7.25 | g |

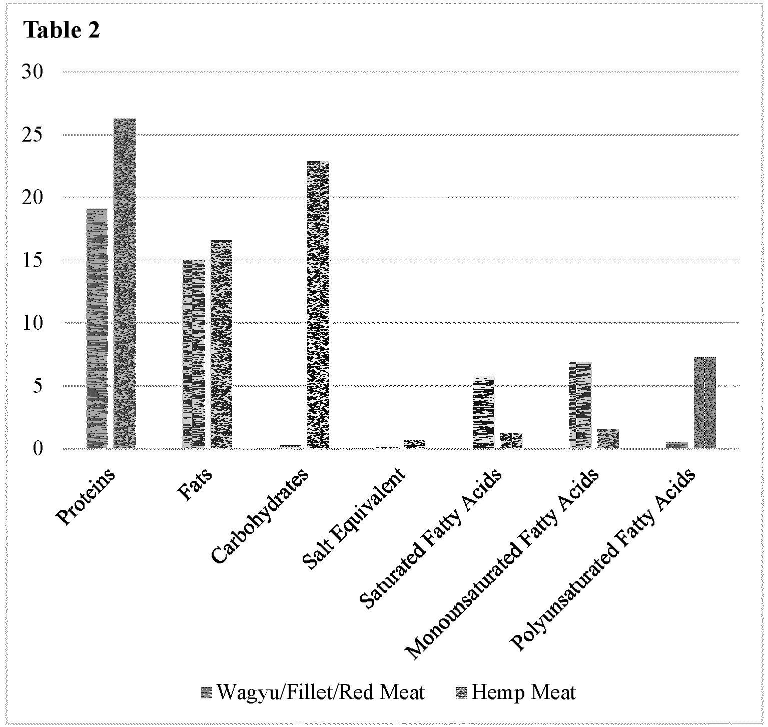

In particular, according to the invention involving the first embodying mode, converting foodstuff data, in which the mouthfeel of predetermined food products has been converted into numerical values, into foodstuff reproduction data enables fabricating new food products in which the mouthfeel of a predetermined food product has been reproduced through a combination of a plurality of mouthfeel fundamentals. What is more, the form of the food being shaped into cubic geometries enables new food products in which the mouthfeel of predetermined food products has been easily reproduced to be made available, without requiring complex forms to shaped.

### Imitation-Meat Food Product Involving Second Embodying Mode

Next, an imitation-meat food product involving a second embodying mode will be described. It should be noted that description regarding items that are in common with the imitation-meat food product involving the first embodying mode will be omitted.

A characteristic of an imitation-meat food product involving the second embodying mode is in that as the vegetable protein source amounting to the principal ingredient, a mixture of a hemp protein source and powdered quinoa is employed.

As stated earlier, hemp has a characteristic grassy odor, bitterness, and astringency, and meanwhile its mouthfeel is absent elasticity and is crusty. Consequently, inasmuch as getting unaltered hemp to gain currency in the market as a meat-replacement product is challenging, mixing it together with other substances is necessary to realize flavors and mouthfeel near that of actual table meat.

Nevertheless, inasmuch as livestock meat extracts for bringing the flavor close to that of table meat, and starch-processed goods for improving mouthfeel are often industrial products, they are not necessarily gentle on the environment and the human body.

That being the case, in the second embodying mode, imitation-meat food products enabling the above-noted issues to be resolved are configured by having the vegetable protein source be one in which quinoa, which is a natural plant, is mixed at a predetermined proportion into a hemp protein source.

Quinoa, like proso millet, foxtail millet, barnyard millet, etc., is a plant that is placed in the minor cereals; mainly manufactured in Peru, Bolivia, and elsewhere in South America, it is a food that has been eaten as a cereal grain since ancient times. Being of extraordinarily high nutritional value, and given that it contains a greater portion of minerals including potassium, magnesium, phosphorus, and iron-than other minor cereals, and that it contains a large amount of linolenic acid, which inhibits the production of cholesterol, it is a foodstuff positioned as a superfood.

What is more, while quinoa does not contain gluten, because it does contain highly viscous starch, it imparts just the right elasticity to the crusty mouthfeel of hemp seeds.

Still, while on the one hand quinoa has a taste free enough of peculiarities that having been washed with water it can be boiled and eaten as is, in that it has a somewhat quinoa-characteristic medicinal odor, quinoa proves inferior in flavor if ingesting a large amount of quinoa alone is attempted.

Therein, in the second embodying mode, imitation-meat food products in diverse variations differing in mixing ratios of hemp protein source and quinoa were fabricated, foodstuff data in which the taste, smell, and mouthfeel of the imitation-meat food products had been converted into numerical values was put together, and optimal mixing proportions were found.

As in Table 3 a portion thereof is indicated, samples, No. 1 to No. 6, were created and imitation-meat food products were fabricated, whereupon evaluations of their smell, taste, and elasticity were carried out.

**Table 3**

| **No.** | **1** | **2** | **3** | **4** | **5** | **6** |
|---|---|---|---|---|---|---|
| **Proportion (Hemp: Quinoa)** | *No quinoa | 5:1 | 4:1 | 3:1 | 2:1 | 4:3 |
| **Hemp Seed** | 20 g | 20 g | 20 g | 20 g | 20 g | 20 g |
| **Hemp Nuts** | 20 g | 20 g | 20 g | 20 g | 20 g | 20 g |
| **Quinoa Powder** | 0 g | 8g | 10 g | 13g | 20 g | 30 g |
| **Vegetable Soup** | 14g | 17g | 18g | 19 g | 21g | 25 g |
| **Smell** | Smells of grassy odor peculiar to hemp | Smells of grassy odor peculiar to hemp | Grassy odor peculiar to hemp, plus smells faintly of beans (cereal) | Grassy odor peculiar to hemp, plus smells faintly of beans (cereal) | Grassy odor peculiar to hemp gone; smells faintly of beans (cereal) | Smells bean-like (cereal-like) |
| **Taste** | Hemp-originating bitterness; post-eating strong astringency left on tongue | Hemp-originating bitterness; post-eating strong astringency left on tongue | Hemp-originating bitterness; post-eating astringency left on tongue | Hemp-originating bitterness; post-eating astringency left on tongue | Hemp-originating bitterness mostly gone; post-eating astringency left on tongue not appearing | Hemp-originating bitterness gone, but cereal sweetness present |
| **Elasticity (Before Steaming)** | In pre-steaming state, almost no stickiness; was state where putting whole together difficult | | | | | In pre-steaming state, strongly sticky; glutinousness in molding |
| **Elasticity (During Tasting)** | No elasticity; crusty and quickly crumbling in mouth powderiness left in mouth | No elasticity; crusty Hemp nuts do not meld; powderiness left in mouth | Slightly less powderiness, but firm without elasticity; stale blandness left in mouth | Powderiness mostly gone, but little meat-like elasticity | Powderiness gone; meat-like elasticity present | Powderiness gone, but slight clinging-to-teeth stickiness and elasticity present |

Sample No. 1 is one into which quinoa was not incorporated, and in which a hemp protein source made up of hemp seeds and hemp nuts was mixed together with vegetable soup and steamed.

Sample No. 2 is one in which the amount of hemp protein source contained was not changed, and into which quinoa powder, being quinoa rendered into powdered form, was incorporated at 20% of the hemp-protein-source content (hemp : quinoa = 5 : 1). It should be noted that the vegetable soup was mixed in so that the mixed-in amount would be a weight that was approximately 35% of the total weight of the hemp and the quinoa. This was likewise the case with Sample No. 3 to Sample No. 6.

In a similar way, Sample No. 3 with quinoa at 25% of the hemp (hemp : quinoa = 4 : 1), Sample No. 4 at 33% (hemp : quinoa = 3 : 1), Sample No. 5 at 50% (hemp : quinoa = 2 : 1), and Sample No. 6 at 75% (hemp : quinoa = 4: 3) were fabricated.

Table 4 and Table 5 present graphs of foodstuff data in which the respective evaluations have been converted into numerical values.

It will be appreciated that evaluations were performed regarding two kinds of smell, hemp-originating hemp odors and quinoa-originating cereal odors, as the smells evaluated.

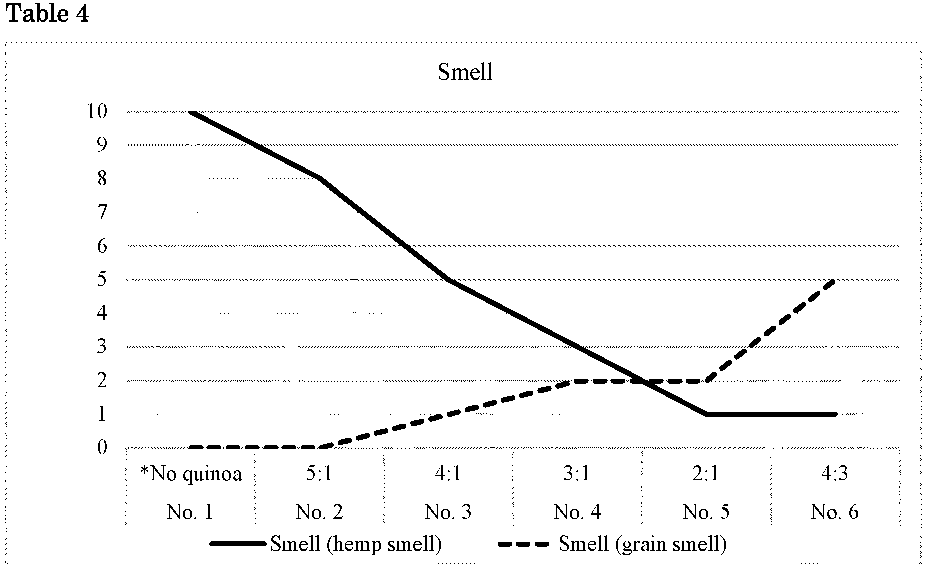

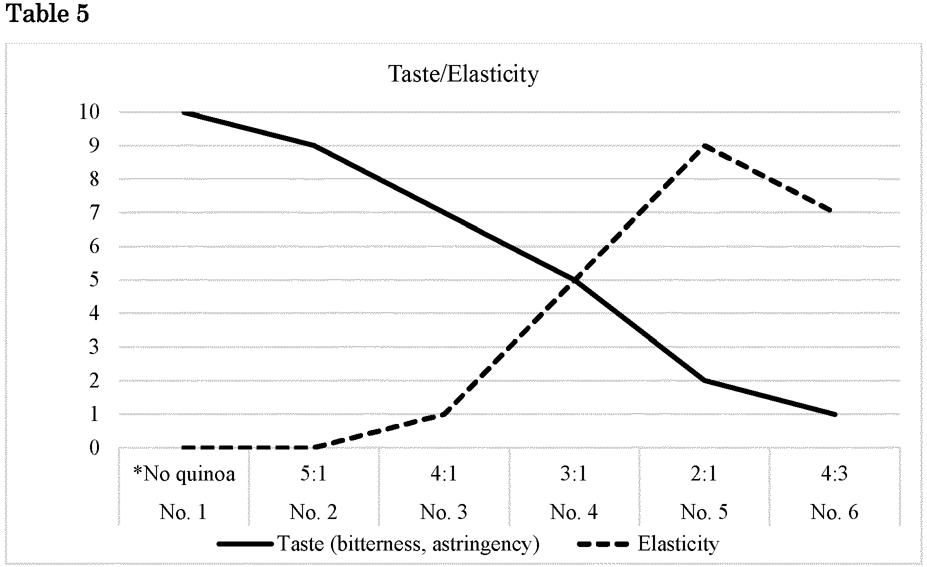

Looking at the outcome for Sample No. 1, which did not contain quinoa: Results were obtained in which along with having the smelly grassy odor peculiar to hemp, hemp-originating bitterness and astringency were sensed. Furthermore, its status was such that, lacking elasticity like that of table meat, and with a powderiness remaining inside the mouth, concluding it to be a foodstuff would be difficult.

In contrast, looking at the results for Sample No. 2, in which quinoa was mixed in at a proportion of 20% of the hemp: Although the elasticity still had not gotten better, the score for the hemp-peculiar smell was somewhat better.

Along the same lines, with Sample Nos. 3 to 5, in which the proportion of quinoa mixed into the hemp protein source 1 had been increased, according as the proportion of quinoa was increased, the grassy smell peculiar to hemp decreased, and the quinoa-originating cereal smell became predominant. Also, it was understood that according as the proportion of quinoa is increased, the hemp-originating bitterness and astringency vanishes, bettering the taste. And as for the mouthfeel, the powderiness disappeared, and with Sample No. 5, a meat-like elasticity was gained.

On the other hand, with Sample No. 6, in which quinoa was mixed in at a proportion of 75% of the hemp, the grassy smell and bitterness peculiar to hemp vanished, but the cereal smell and sweetness from quinoa origins became predominant. Further, with a strong stickiness, glutinousness occurred during molding, and meanwhile there was a stickiness to the sample such as to cling slightly to the teeth when being chewed. In short, because making the amount of quinoa too great leads to the features that quinoa has becoming predominant, resulting in loss of table-meat sort of flavors and mouthfeel, maintaining appropriate mixing proportions proves to be crucial.

In this way, putting together in advance foodstuff data in which the smell, taste, and elasticity of the fabricated imitation-meat food product have been converted into numerical values, and mixing in quinoa at a mixing proportion determined on the basis of the foodstuff data put together in advance enable fabricating an imitation-meat food product of flavor and mouthfeel that is near that of table meat.

In particular, in the second embodying mode, because into the hemp foodstuff, which has a characteristic odor and mouthfeel, is mixed quinoa, which likewise has a characteristic odor and mouthfeel, mixing them together at a mixing ratio such that the good points of both are taken advantage of and at the same time their bad points are canceled out proves to be crucial.

Therefore, by determining, on the basis of foodstuff data in which the taste, smell, and mouthfeel of an imitation-meat food product to be created are converted into numerical values, the proportion of quinoa that is mixed in with hemp seeds, an imitation-meat food product that, taking advantage of the features of hemp and quinoa, has flavors and mouthfeel near that of actual table meat can be obtained.

It should be noted that in the second embodying mode, likewise as with the first embodying mode, a plurality of mouthfeel fundamentals that furnish differing mouthfeels can be combined to create an imitation-meat food product having a complex mouthfeel like that of actual table meat. Namely, by preparing vegetable protein sources of a plurality of types in which the mixing proportions of hemp and quinoa have been varied, and as illustrated in Figs. 4 to 9, molding so as to dispose one vegetable protein source in one section and another vegetable protein source in the remaining section, an imitation-meat food product having sectionally differing mouthfeels may be made available.

In other aspects as well, the second is likewise as with the first embodying mode.

Furthermore, also in the second embodying mode, the imitation-meat food product can be fabricated by the same sort of fabricating method as with the first embodying mode.

Namely, an imitation-meat food product can be fabricated by: a step of preparing a predetermined amount of hemp protein source; a step of weighing out powdered quinoa so as to be at a predetermined proportion with respect to the predetermined amount of hemp protein source; a step of mixing the hemp protein source with the weighed-out powdered quinoa to create a vegetable protein source; a step of kneading the vegetable protein source together with water, vegetable soup, or other moisture content to create an ingredient mixture; a step of heat-coagulating the kneaded ingredient mixture; and a step of molding the heat-coagulated mixture. Then, when the powdered quinoa is weighed out so as to be at a predetermined proportion with respect to the hemp protein source, the weighing-out is performed with reference to foodstuff data put together in advance, so that just the right flavor and elasticity will be obtained.

It should be understood that whereas an example in which both hemp seed and hemp nuts are utilized as a hemp protein source has been illustrated, there would be no concern in the source being one in which only either hemp seeds or hemp nuts are utilized.

Further, while powdered quinoa has been exemplified as being what is mixed in with the hemp protein source, there would be no concern in it being granulated quinoa in which a suitable grain size is left that is mixed in.

### Action and Effect of Invention Involving Second Embodying Mode

According to the invention involving the second embodying mode, an imitation-meat food product can be made available that employs a hemp protein source whose burden on the environment is minimal and that is of high nutritional value, and meanwhile that, with the elasticity and flavors that quinoa possesses, can compensate for the bitterness and meagerness of mouthfeel peculiar to hemp, and that when chewed is able to yield flavors and mouthfeel near that of natural meat.

It should be understood that while the present embodying modes and modification examples have been described, as other modes of embodying the present invention, the above-described embodying modes and modification examples may be combined in their entireties or in part. Moreover, modes of embodying the present invention are not limited to the above-described embodying modes, but various changes, substitutions, and modifications may be made within a range in which the gist of the technical concepts of the present invention is not departed from. Furthermore, if due to advances in technology or separate technology that is derived, the technical concepts of the present invention can be realized in a separate way, they may be embodied utilizing that method. Accordingly, the scope of the claims comprehends all embodying modes that can fall within the scope of the technical concepts of the present invention.

### INDUSTRIAL APPLICABILITY

An imitation-meat food product and a method of its fabrication involving the present invention are applicable to provision in restaurants and to provision in various modes, including marketing as ready-made processed food products in supermarkets and the like.

### DESCRIPTION OF REFERENCE MARKS

- **1:**: Food-product fabrication apparatus
- **3:**: Imitation meat
- **13:**: Water tank
- **16:**: Tube
- **17:**: Foodstuff tank
- **17a-d:**: Fundamentals
- **18:**: Mixing means
- **19:**: Output nozzle
- **20:**: Extruding means
- **21:**: Stirring means
- **22:**: Die frame
- **23:**: Recess
- **24:**: Enzyme cartridge
- **25:**: Downstream end
- **31:**: Fatty area
- **32:**: Lean area

## Claims

1. An imitation-meat food with a vegetable protein source being the principal ingredient, the imitation-meat food **characterized in that**:
the vegetable protein source is constituted from a mixture of a hemp protein source made up of hemp seeds and/or hemp nuts, and powdered quinoa mixed in so as to be at a predetermined proportion with respect to the hemp protein source.

2. The imitation-meat food set forth in claim 1, wherein the proportion of powdered quinoa mixed in with the hemp protein source is determined based on foodstuff data in which the taste, smell, and mouthfeel of created imitation-meat food have been converted into numerical values.

3. A method of fabricating an imitation-meat food with a vegetable protein source being the principal ingredient, the method of fabricating an imitation-meat food **characterized in**:
a step of preparing hemp protein source made up of hemp seeds and/or hemp nuts;
a step of weighing out powdered quinoa so as to be at a predetermined proportion with respect to the hemp protein source;
a step of mixing the weighed-out quinoa together with, and kneading it into, the hemp protein source; and
a step of heat-coagulating the mixed-together hemp protein source and powdered quinoa.
